(19) Europäisches Patentamt
European Patent Office
Office européen des brevets

(11) Veröffentlichungsnummer: **0 049 911**
**B1**

(12) # EUROPÄISCHE PATENTSCHRIFT

(45) Veröffentlichungstag der Patentschrift: **15.02.84**

(51) Int. Cl.³: **C 07 F 9/40** // C07C45/55, C07C47/575, C07C47/55

(21) Anmeldenummer: **81110809.1**

(22) Anmeldetag: **09.08.80**

(60) Veröffentlichungsnummer der früheren Anmeldung nach Art. 76 EPÜ:

(54) **Verfahren zur Herstellung von alpha-Hydroxybenzylphosphonsäureestern.**

(30) Priorität: **22.08.79 DE 2934034**

(43) Veröffentlichungstag der Anmeldung: **21.04.82 Patentblatt 82/16**

(45) Bekanntmachung des Hinweises auf die Patenterteilung: **15.02.84 Patentblatt 84/7**

(84) Benannte Vertragsstaaten: **BE CH DE FR GB IT LI NL**

(56) Entgegenhaltungen:
**EP-A-0 019 719**

**CHEMISCHE BERICHTE, Band 103, Nr. 9, 1970, Seiten 2984-2986, L. HORNER et al.: "Notiz über die reduktive Umwandlung von Carbonsäuren in ihre Aldehyde".**
**COMPTES RENDUS, Hebdomadaires, Des séances de l'académie des sciences, Tome deux cent soixante-seizième, Série C: Sciences Chimiques, troisième partie: mai-juin 1973, Seiten 1807-1810 G. STURTZ et al.: "Chimie Organique. Action du n-butyllithium sur quelques dialkyl et bis-diméthylamido phosphates de benzyle substitué. Réarrangement phosphatephosphonate".**
**CHEMICAL ABSTRACTS, Band 69, Nr. 17, 21. Oktober 1968, Seite 6307, Nr. 67465y, Columbus, Ohio, USA V. ABRAMOV et al.: "Reactions of dialkyl phosphonates**

(73) Patentinhaber: **BAYER AG, Zentralbereich Patente, Marken und Lizenzen, D-5090 Leverkusen 1, Bayerwerk (DE)**

(72) Erfinder: **Hoffmann, Hellmut, Prof. Dr., Tersteegenweg 17, D-5600 Wuppertal 1 (DE)**

(56) Entgegenhaltungen: (Fortsetzung)
**with aldehydes and ketones. XXVIII. Esters of alpha-hydroxy-beta-chloroethylphosphonic acids and alpha,beta-epoxyethylphosphonic acids".**

**Die Akte enthält technische Angaben, die nach dem Eingang der Anmeldung eingereicht wurden und die nicht in dieser Patentschrift enthalten sind.**

**EP 0 049 911 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von α-Hydroxybenzylphosphonsäureestern

Die Erfindung betrifft ein neues Verfahren zur Herstellung von α-Hydroxy-benzyl-phosphonsäureestern.

Es ist bekannt, dass man bestimmte substituierte Benzaldehyde, wie z.B. 4-Fluor-3-phenoxybenzaldehyd, erhält, wenn man Benzylhalogenide, wie z.B. 4-Fluor-3-phenoxy-benzylbromid, mit Hexamethylentetramin umsetzt und das Produkt dieser Umsetzung mit Säuren erhitzt (vergleiche DE-OS 2 709 264).

Bei dieser Synthesemethode erhält man jedoch eine Reihe von substituierten Benzaldehyden nur in geringen Ausbeuten; unbefriedigend sind die Ausbeuten auch bei der Synthese der als Ausgangsstoffe zu verwendenden Benzyl-halogenide, beispelsweise durch Umsetzung von Phenoxy-toluolen mit N-Brom-succinimid.

In Comptes Rendus Hebdomadaires, Band 276, Serie C – 1807 ff. wird die basische Hydrolyse eines in α-Stellung alkylierten Oxanions eines Phosphoramids beschrieben. In einem Reaktionsschema wird dabei die intermediäre Bildung von Benzaldehyd postuliert. Dieser Literaturstelle kann jedoch kein Hinweis darauf entnommen werden, ob und unter welchen Bedingungen aus α-Hydroxyphosphonestern Aldehyde gebildet werden.

In Chem. Berichte, Band 103, S. 2984–2986 wird erwähnt, dass α-Ketophosphonsäureester mit Hilfe von Natriumborhydrid zu den α-Hydroxyphosphonestern reduziert werden und daraus in alkalischem Medium die entsprechenden Aldehyde freigesetzt werden können. Entscheidend für eine gute Aldehydausbeute scheint jedoch zu sein, dass die α-Hydroxyphosphonsäureester aus den α-Ketophosphonsäureestern in Gegenwart eines hohen Überschusses an Natriumborhydrid hergestellt werden. Wird mit einer geringeren Menge Natriumborhydrid gearbeitet, sinkt die Ausbeute.

Die vorliegende Erfindung betrifft demgegenüber
ein Verfahren zur Herstellung von α-Hydroxybenzyl-phosphonsäureestern der Formel II

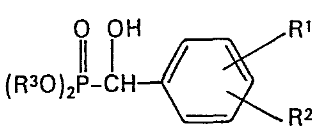

(II)

in welcher
$R^1$ für Wasserstoff oder Halogen steht und
$R^2$ für Halogen oder gegebenenfalls halogensubstituiertes Phenoxy steht und
$R^3$ einzeln für Alkyl oder Phenyl oder zusammen für Alkandiyl (Alkylen) stehen,
das dadurch gekennzeichnet ist, dass man Benzoylphosphonsäureester der Formel III pro Mol

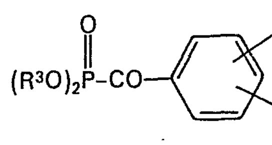

(III)

in welcher
$R^1$, $R^2$ und $R^3$ die oben angegebene Bedeutung haben,
mit 0,2 bis 0,5 Mol Natrium- oder Kaliumborhydrid
gegebenenfalls in Gegenwart eines Puffermittels in einem Zweiphasensystem aus Waser und einem mit Wasser nicht mischbaren Lösungsmittel bei Temperaturen zwischen −20 und +50°C umsetzt.

Die α-Hydroxyphosphonsäureester der Formel II

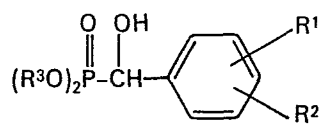

(II)

in welcher
$R^1$ für Wasserstoff oder Fluor in p-Stellung steht,
$R^2$ für gegebenenfalls fluorsubstituiertes Phenoxy in m-Stellung oder falls $R^1$ für Fluor steht, für Brom steht,
$R^3$ für $C_{1-4}$-Alkyl, Phenyl oder zusammen für 2,2-Dimethyl-propan-1,3-diyl steht,
sind neu.

Überraschenderweise können nach dem erfindungsgemässen Verfahren 1) die α-Hydroxyphosphonsäureester wesentlich einfacher, kostengünstiger und in besseren Ausbeuten als nach bekannten Verfahren hergestellt werden. Auch den so hergestellten α-Hydroxybenzylphosphonsäureestern lassen sich besonders vorteilhaft die entsprechenden Aldehyde herstellen.

Verwendet man als Ausgangsstoffe beispielsweise 3-Phenoxybenzoyl-phosphonsäuredimethylester und Natriumtetrahydridoborat und bei der Umsetzung des hierbei gebildeten α-Hydroxy-3-phenoxybenzyl-phosphonsäuredimethylester Kaliumhydroxid als Reaktionskomponente, so kann der Reaktionsablauf nach folgendem Reaktionsschema skizziert werden:

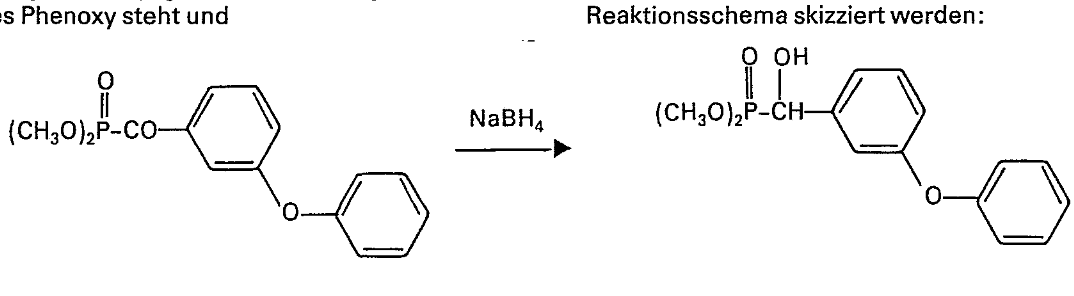

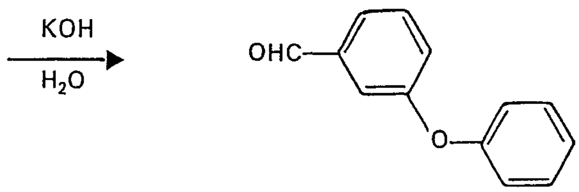


Das erfindungsgemässe Verfahren wird im Zweiphasensystem aus Wasser und mit Wasser nicht mischbaren Lösungsmitteln, wie z.B. Hexan, Heptan, Cyclohexan, Methylenchlorid, Chloroform, Ethylenchlorid, Diethylether, Toluol oder Xylol durchgeführt.

Puffermittel, welche beim erfindungsgemässen Verfahren zum Halten des pH-Wertes bei ca. 6-7 verwendet werden können, sind z.B. Natriumhydrogenphosphat und Kaliumhydrogenphosphat.

Als Reduktionsmittel wird Natriumtetrahydridoborat (Natriumboranat) vorzugsweise verwendet.

Die Reaktionstemperatur wird zwischen −20 und +50°C, vorzugsweise zwischen −10 und +30°C gehalten. Das Verfahren wird im allgemeinen bei Normaldruck durchgeführt.

Zur Durchführung werden auf 1 Mol Benzoylphosphonsäureester der Formel (III) zwischen 0,2 und 0,5 Mol, vorzugsweise zwischen 0,25 und 0,4 Mol Natrium- oder Kaliumborhydrid eingesetzt.

Das Hydrid, vorzugsweise Natriumtetrahydridoborat, wird in einem Zweiphasengemisch aus Wasser und einem mit Wasser nicht mischbaren Lösungsmittel vorgelegt und der Benzoyl-phosphonsäureester, gegebenenfalls in einem mit Wasser nicht mischbaren Lösungsmittel gelöst, eindosiert. Das Reaktionsgemisch wird bis zum Reaktionsende gerührt, die organische Phase anschliessend abgetrennt, getrocknet, durch Vakuumdestillation vom Lösungsmittel befreit und «andestilliert», d.h. einige Zeit bei geringfügig erhöhter Temperatur evakuiert.

Die Produkte der Formel (II) werden so in öliger Form relativ rein erhalten.

Die als Ausgangsverbindungen zu verwendenden Benzoyl-phosphonsäureester sind durch Formel (III) definiert.

Als Beispiele für Verbindungen der Formel (III) seien genannt: 3-Brom-4-fluor-benzoyl-, 3-Phenoxy-benzoyl-, 4-Fluor-3-phenoxy-benzoyl-,
3-(4-Fluorphenoxy)-benzoyl- und
4-Fluor-3-(4-fluor-phenoxy)-benzoyl-phosphonsäure-dimethylester und -diethylester sowie
2-Oxo-2-(3-phenoxy-benzoyl)-,
2-Oxo-2-(4-fluor-3-phenoxybenzoyl)-,
2-Oxo-2-(3-(4-fluor-phenoxy)-benzoyl)- und
2-Oxo-2-(4-fluor-3-(4-fluor-phenoxy)-benzoyl)-5,5-dimethyl-1,3,2-dioxaphosphorinan.

Die Benzoylphosphonsäureester sind zum Teil Gegenstand einer nicht vorveröffentlichten Patentanmeldung (vergl. EP-A-20 988). Man erhält sie durch Umsetzung von Phosphorigsäureester der Formel V

$$(R^3O)_2P-OR^4 \quad (V)$$

in welcher
$R^3$ die oben angegebene Bedeutung hat und
$R^4$ für Methyl oder Ethyl steht,

mit Benzoesäurehalogeniden der Formel VI

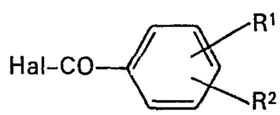

(VI)

in welcher
$R^1$ und $R^2$ die oben angegebene Bedeutungen haben und
Hal für Fluor, Chlor oder Brom, vorzugsweise für Chlor steht,
bei Temperaturen zwischen 0 und 150°C, vorzugsweise zwischen 20 und 100°C.

In den Formeln (V) und (VI) haben $R^1$, $R^2$ und $R^3$ die gleichen bevorzugten Bedeutungen wie in Formel (II).

Als Beispiele für die Phosphorigsäureester der Formel (V) seien Trimethylphosphit, Triethylphosphit und 2-Ethoxy-5,5-dimethyl-1,3,2-dioxaphosphorinan genannt.

Als Beispiele für die Benzoesäurehalogenide der Formel (IV) seien genannt: 3-Brom-4-fluorbenzoylchlorid, 3-Phenoxy-benzoylchlorid, 4-Fluor-3-phenoxy-benzoyl-chlorid, 3-(4-Fluorphenoxy)-benzoylchlorid und 4-Fluor-3-(4-fluorphenoxy)-benzoylchlorid. Diese Verbindungen sind zum Teil bekannt oder lassen sich analog zu bekannten Verfahren herstellen.

So ist 3-Phenoxy-benzoylchlorid bekannt aus GB-PS 1 052 390.

Die noch nicht in der Literatur beschriebenen fluor-substituierten Phenoxybenzoesäurehalogenide erhält man, wenn man gemäss nachstehendem Reaktionsschema entsprechend substituierte Brombenzoesäuren (bzw. deren Alkalisalze) mit Alkaliphenolaten in Gegenwart eines Katalysators, wie z.B. Kupferoxid, und unter Verwendung von Phenol als Verdünnungsmittel bei Temperaturen zwischen 100 und 250°C, vorzugsweise zwischen 150 und 200°C umsetzt (vergleiche die nicht vorveröffentlichte EP-A-17 882) und die hierbei gebildeten Phenoxybenzoesäuren mit einem Halogenierungsmittel, wie z.B. Thionylchlorid, bei Temperaturen zwischen 20 und 100°C umsetzt:

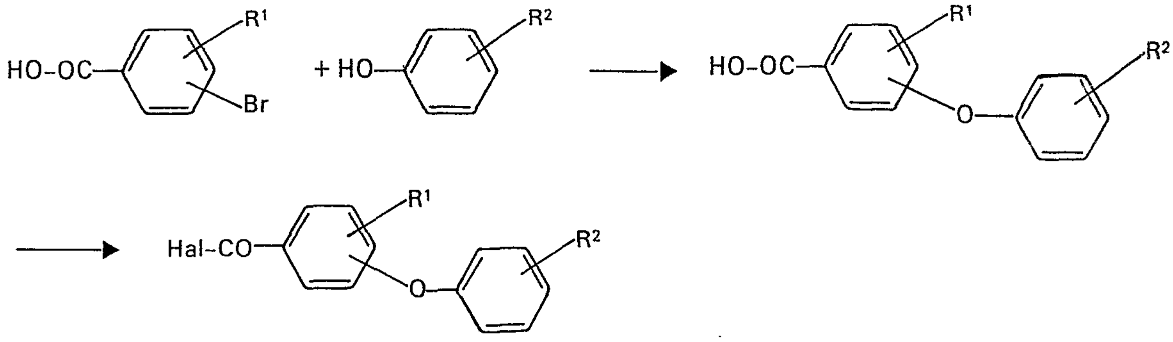

Die α-Hydroxybenzylphosphonsäureester der Formel II sind zum Teil neu. Als Beispiele für die neuen α-Hydroxybenzylphosphonsäureester (II) seien genannt:

α-Hydroxy-3-phenoxy-benzyl-,
α-Hydroxy-3-brom-4-fluorbenzyl-,
α-Hydroxy-4-fluor-3-phenoxy-benzyl-,
α-Hydroxy-3-(4-fluor-phenoxy)-benzyl- und
α-Hydroxy-4-fluor-3-(4-fluor-phenoxy)-benzylphosphonsäuredimethylester und -diethylester sowie
2-Oxo-2-(α-hydroxy-3-phenoxy-benzyl)-,
2-Oxo-2-(α-hydroxy-4-fluor-3-phenoxy-benzyl)-,
2-Oxo-2-(α-hydroxy-3-(4-fluor-phenoxy)-benzyl)- und
2-Oxo-2-(α-hydroxy-4-fluor-3-(4-fluor-phenoxy)-benzyl-5,5-dimethyl-1,3,2-dioxaphosphorinan.

Die α-Hydroxybenzylphosphonsäureester werden verwendet, um daraus durch Behandlung mit wässrigen Alkali- oder Erdalkalihydroxidlösungen substituierte Benzaldehyde herzustellen.

Die nach dem erfindungsgemässen Verfahren herzustellenden Benzaldehyde können als Zwischenprodukte zur Herstellung von Schädlingsbekämpfungsmitteln verwendet werden (Vgl. DE-OS 2 621 433).

Beispiel 1:

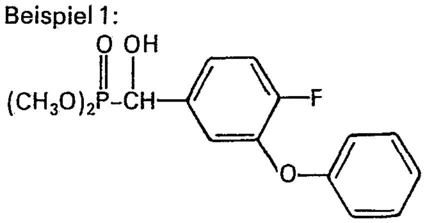

Eine Lösung von 130 g (0,4 Mol) 4-Fluor-3-phenoxy-benzoyl-phosphonsäure-dimethylester in 200 ml Methylenchlorid wird zu einer auf 0°C abgekühlten, intensiv gerührten Mischung aus 4,8 g (0.12 Mol) Natriumtetrahydridoborat, 200 ml Methylenchlorid und 400 ml Wasser tropfenweise gegeben. Das Reaktionsgemisch wird 90 Minuten bei 0°C gerührt, dann trennt man die organische Phase ab und extrahiert die wässrige Phase mit 200 ml Methylenchlorid. Die vereinigten Methylenchlorid-Phasen werden getrocknet, filtriert und das Lösungsmittel wird sorgfältig abdestilliert. Man erhält 120 g (92% der Theorie) α-Hydroxy-4-fluor-3-phenoxy-benzyl-phosphonsäure-dimethylester als zähflüssiges Öl.

Elementaranalyse:

Ber.: C 55,2% H 4,9% P 9,5%
Gef.: C 55,0% H 4,9% P 9,4%

Beispiel 2: (Herstellung der Ausgangsverbindung)

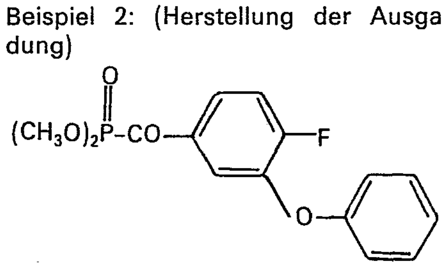

62 g Trimethylphosphit werden bei einer Innentemperatur von 60 bis 65°C zu 126 g (0,5 Mol) 4-Fluor-3-phenoxy-benzoylchlorid tropfenweise gegeben. Das Reaktionsgemisch wird eine Stunde bei 80°C gerührt und evakuiert. Man erhält 158 g (97% der Theorie) 4-Fluor-3-phenoxy-benzoyl-phosphonsäure-dimethylester als zähflüssiges Öl.

Elementaranalyse:

Ber.: C 55,6% H 4,3% P 9,6%
Gef.: C 55,0% H 4,3% P 9,7%

Beispiel 3:

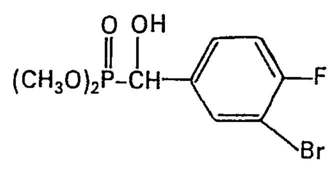

Eine Lösung von 94 g (0,3 Mol) 3-Brom-4-fluor-benzoyl-phosphonsäure-dimethylester in 100 ml Methylenchlorid wird zu einer auf 0°C gekühlten, intensiv gerührten Mischung aus 5 g Natriumtetrahydridoborat, 150 ml Methylenchlorid und 300 ml Wasser tropfenweise gegeben. Das Reaktionsgemisch wird 90 Minuten bei 0°C gerührt, dann trennt man die organische Phase ab und extrahiert die wässrige Phase mit 200 ml Methylenchlorid. Die vereinigten Methylenchlorid-Phasen werden getrocknet und filtriert. Vom Filtrat wird das Lösungsmittel unter vermindertem Druck sorgfältig abdestilliert. Nach Anreiben mit Ligroin kristallisiert das Produkt. Man erhält 75 g (80% der Theorie) α-Hydroxy-3-brom-4-fluor-benzyl-phosphonsäure-dimethylester vom Schmelzpunkt 91–93°C.

Beispiel 4: (Herstellung der Ausgangsverbindung)

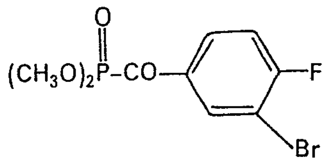

38 g Trimethylphosphit werden bei einer Innentemperatur zwischen 35 und 40°C zu einer Lösung von 72 g (0,3 Mol) 3-Brom-4-fluor-benzoylchlorid in 100 ml Methylenchlorid gegeben und das Reaktionsgemisch wird drei Stunden gerührt. Dann wird das Lösungsmittel unter vermindertem Druck sorgfältig abdestilliert. Man erhält 92 g (98% der Theorie) 3-Brom-4-fluor-benzoyl-phosphonsäure-dimethylester vom Siedepunkt 122°C/0,01 mBar.

**Patentanspruch**

1. Verfahren zur Herstellung von α-Hydroxybenzylphosphonsäureestern der Formel (II)

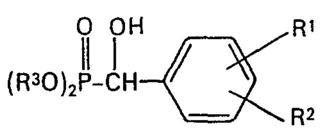

(II)

in welcher
$R^1$ für Wasserstoff oder Halogen steht,
$R^2$ für Halogen oder gegebenenfalls halogensubstituiertes Phenoxy steht und die Reste
$R^3$ einzeln für Alkyl oder Phenyl oder zusammen für Alkandiyl stehen,
indem man Benzoylphosphonsäureester der Formel (III)

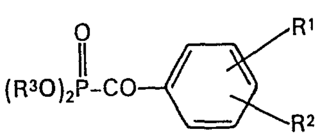

(III)

in welcher
$R^1$–$R^3$ die oben angegebene Bedeutung haben,
mit Natrium- oder Kaliumborhydrid
gegebenenfalls in Gegenwart eines Puffermittels bei Temperaturen von −20 bis +50°C umsetzt, das dadurch gekennzeichnet ist, dass pro Mol Benzoylphosphonester der Formel (II) 0,2 bis 0,5 Mol Hydrid eingesetzt werden und in einem Zweiphasengemisch aus Wasser und einem mit Wasser nicht mischbaren Lösungsmittel gearbeitet wird.

**Claim**

1. Process for the preparation of α-hydroxybenzylphosphonic acid esters of the formula (II)

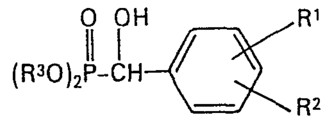

(II)

in which
$R^1$ represents hydrogen or halogen,
$R^2$ represents halogen or optionally halogen-substituted phenoxy and
the radicals $R^3$ individually represent alkyl or phenyl or together represent alkanediyl,
by reacting benzoylphosphonic acid esters of the formula (III)

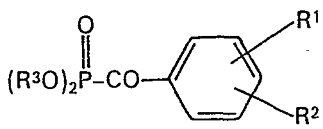

(III)

in which
$R^1$–$R^3$ have the meaning indicated above, with sodium hydridoborate or potassium hydridoborate, if appropriate in the presence of a buffer at temperatures of −20 to +50°C, which process is characterised in that per mol of benzoylphosphonic esters of the formula (II) 0.2 to 0.5 mol of hydride are used and the reaction is carried out in a two-phase mixture of water and a water-immiscible solvent.

**Revendication**

1. Procédé de préparation d'esters d'acides α-hydroxybenzylphosphoniques de formule (II)

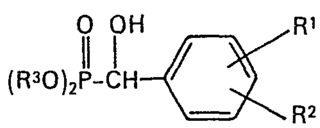

(II)

dans laquelle
$R^1$ représente de l'hydrogène ou de l'halogène,
$R^2$ de l'halogène ou un phénoxy éventuellement halo-substitué et les radicaux
$R^3$ représentent individuellement un alcoyle ou phényle ou conjointement un alcane diyle, dans lequel on fait réagir des esters d'acides benzoylphosphoniques de formule (III)

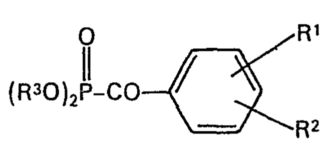

(III)

dans laquelle
$R^1$–$R^3$ ont la signification indiquée plus haut, avec

du borohydrure de sodium ou de potassium éventuellement en présence d'un agent tampon à des températures de −20 à +50°C, caractérisé en ce qu'on utilise par mole d'ester benzoylphosphonique de formule (II) 0,2 à 0,5 mole d'hydrure et en ce qu'on opère dans un mélange à deux phases composé d'eau et d'un solvant non miscible avec l'eau.